# EUROPEAN PATENT APPLICATION

(11) **EP 1 457 216 A2**
(43) Date of publication of application: **15.09.2004**
(21) Application number: 04425085.0
(22) Date of filing: 10.02.2004
(51) Int. Cl.: A61M 1/00

(54) **Filtering device with bypass**

(30) Priority: 13.03.2003 IT BS20030027
(71) Applicant: Physioplant S.r.l., 25030 Roncedelle (Brescia) (IT)
(72) Inventor: Rizzo, Rosario, 25030 Roncadelle (Brescia) (IT)
(74) Representative: Sangiacomo, Fulvia

(57) **Abstract**

The invention concerns a filtering device for separating solid parts from fluid parts aspired by means of an aspiration unit. It comprises a diverter means (29) movable between a first position, where an entrance passage (20) for the aspired material is placed in communication with a channel in a fluid communication with an evacuation passage, and a second position, where the entrance passage (20) for the aspired material is placed in communication with a filtering chamber (14) also connected to the evacuation passage.

## Description

### Filed of the Invention

The present invention refers to a filtering device to be connected to an aspirator and intended to separate and trap solid parts from fluid parts.

### State of the Art

In surgical operations or treatments, wherever fluid substances have to be removed from the treatment area, the use is frequent of aspirators that essentially consist of a pipe having an aspiration piece at a proximal end that can be connected to a vacuum unit at the distal end.

Usually, such aspirators aspire and remove everything within the reach of the aspiration piece, meaning fluid parts and solid parts promiscuously. During the course of some operations however, for example, but not limited to, dental surgery, it is best to separate the solid parts, normally bone fragments, from the fluid parts such as saliva, blood, etc., in order to recover the former and use these again to favour regeneration of the same patient's operated bone.

When, as is commonly the case, aspiration occurs promiscuously with just one aspirator, the risk exists of the solid parts, meaning the bones, being lost or being altered by the fluid parts and, in any case, separation for their recovery must be done downstream at a later stage in not exactly sterile conditions. Being thus separated therefore, the solid parts can be polluted and also dry up, jeopardising their use.

To separately aspire and collect the solid parts from the fluid parts therefore, currently two specific aspirators are used, one for the fluid parts and the other for the solid parts; this however has evident drawbacks of both a practical and economic nature because two machines are needed and must be managed.

### Objects and Summary of the invention

One object of this invention is to overcome the above problems and drawbacks and thus allow the recovery of bone fragments, or in any case solid parts, during the course of a surgical operation, in sterile conditions, without subsequent handling and leaving the solid parts unaltered for their subsequent use.

Another object of the invention is to provide a device able to aspire fluid parts and solid parts selectively using a single aspiration source and, advantageously, already-available connector and auxiliary elements, meaning without the need to change the usual configuration of the aspirators on the market.

Yet another object of the invention is to provide a device able to perform two alternate operations and to filter and trap the solid parts, according to need, under the easy control of just one operator.

Yet a further object of the invention is to provide a simple and economical single-use type of device for the above-mentioned embodiment use.

Said purposes are achieved, according to the invention, with a filtering device in accordance with claim 1.

### Brief description of the drawings

The invention will also be illustrated in more detail later on in this description and making reference to the approximate and not limitative drawings, where:

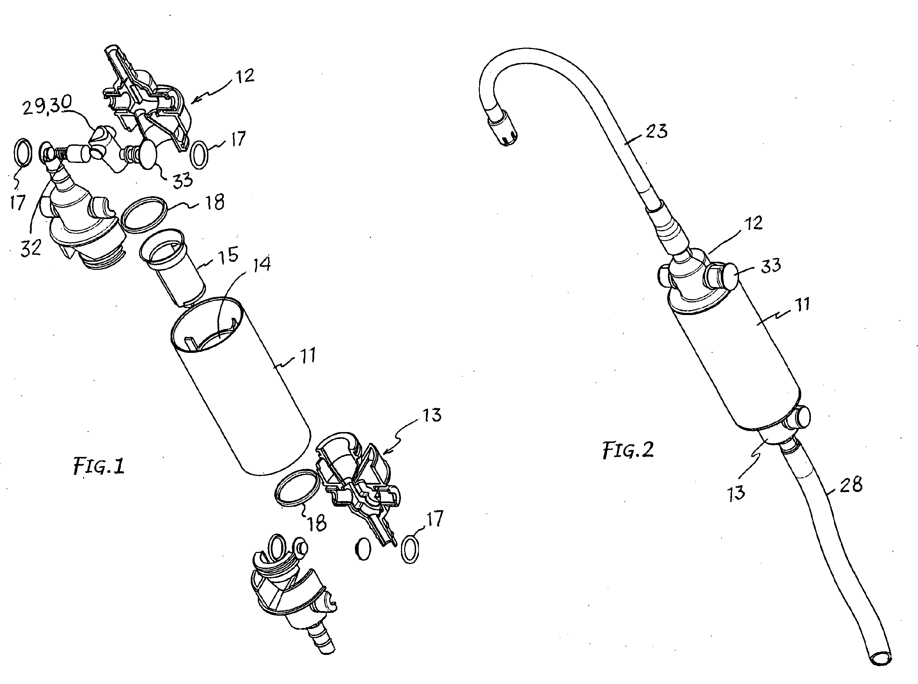

Fig. 1 is an exploded view of the components of the device;
Fig. 2 is a perspective view of the assembled device complete with aspiration piece and a pipe to be connected to an aspirator;
Fig. 3 is a longitudinal section of the device in Fig. 2 in a first operating position;
Fig. 4 is a part of the device in Fig. 3 in a second operating position and corresponding to the section according to the arrows A-A- on Fig. 5; and
Fig. 5 is a view of the end of the device in the position in Fig. 4.

### Detailed description of the invention

The device shown comprises a hollow body 11 with a first head connection 12 at one of its ends and a second head connection 13 at the opposite end, said parts being preferably made of plastic material.

The hollow body 11 is configured to define a filtering chamber 14 in which is housed a basket filter 15 and, around said chamber, one or more longitudinal channels 16.

Structurally, the first and the second connections 12, 13 can be different, but more preferably and as shown in the drawings they are identical as this is more advantageous from a construction viewpoint inasmuch as it can be achieved using the same mould.

Thus, for example, each head connection 12, 13 can be formed of two complementary casings, facing each other and joined together with the aid of ring-shaped fasteners 17. Each connection 12, 13 is fastened at the respective end of the body 11 and features an internal part 12', 13' associated by sealing with the filtering chamber 14 with the interposition of at least one seal 18. The filter 15 in the filtering chamber 14 is placed between the internal parts 12', 13' of the two connections coupled with the chamber itself.

More specifically, the first connection 12 features an entrance anti-chamber 19 that communicates, on the one side, with an entrance passage 20 and, on the other, with an entrance conduit 21 communicating with the filtering chamber 14 and with an entrance compartment 22, in turn communicating with the longitudinal channel/s 16 in the body 11.

The entrance passage 20 has the shape of a hose connection 20' to which at least one aspiration piece 23 can be connected which can be of any kind while the entrance conduit 21 is defined by the inner portion 12' of the connection 12 associated with the filtering chamber 14.

Similarly, the second connection 13, when this is identical to the first connection 12, features an exit anti-chamber 24 that communicates, on the one side, with an evacuation passage 25 and, on the other, with an exit conduit 26 communicating with the filtering chamber 14 and with an exit compartment 27 in turn communicating with the longitudinal channel/s 16 in the body 11. The evacuation passage 25 has the shape of a hose connection 25' to which an aspiration pipe 28 is attached that can be connected to an aspirator unit - not represented, while the exit pipe 26 is defined by the internal part 13' of the connection 13 associated with the filtering chamber 14.

To the first connection 12, at the level of the entrance anti-chamber 19, is associated a diverter means 29 movable in two different positions to put alternatively into communication the entrance passage 20 with the entrance compartment 22, and thus with the longitudinal channels 16 in the body 11, or with the entrance conduit 21, and thus with the filtering chamber 14.

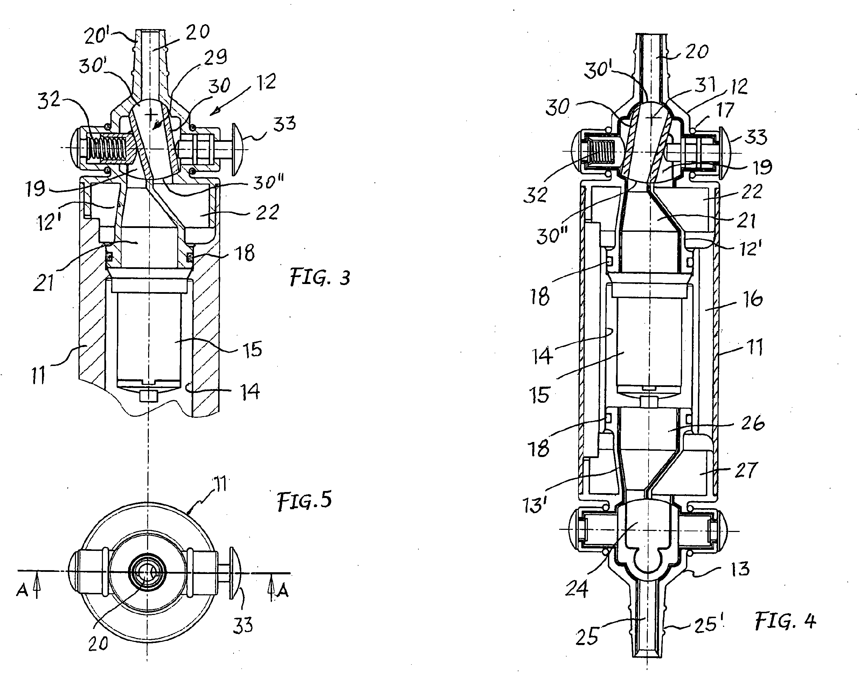

In the example shown, the diverter 29 consists of a tubular piece 30 movable around an oscillation axis 31 and having an inlet 30' permanently in line with the entrance passage 20 and an outlet 30" which in a first position is in line with the entrance compartment 22 and in a second position is in line with the entrance conduit 21. As Figures 3 and 4 show, the oscillation of the diverter means 29 is caused in one direction by a spring 32 that tends to keep it in said first position and in the other direction by a button 33 for placing the diverter in said second position.

In the second connection, when this is the same as the first, no diverter is envisaged or needed, and in place of the spring and button, blind caps are fitted.

In actual fact, the entrance compartment 22 in the first connection 12 communicates with the exit compartment 27 in the second connection 13 through the longitudinal channel/s 16 in the body 11 and the entrance conduit 21 in the first connection 12 communicates with the exit conduit 26 in the second connection 13 through the chamber 14 containing filter 15. Aspirator unit aspiration reaches piece 23 through one or other of these routes depending on the position of the diverter 29.

As long as the diverter means 29, pushed by spring 32, is in first position - Fig. 3 - everything sucked up by piece 23 passes through the diverter and from this is conveyed to the evacuation passage 25 through the entrance compartment 22 the longitudinal channels 16 and the exit compartment 27. When, on the other hand, the diverter means 29 is moved to the second position - Fig. 4 - by means of button 33, the material aspired by piece 23 is conveyed to the filtering chamber 14 where the solid or larger parts are trapped by filter 15 and separated from any fluid parts which are eliminated through the evacuation passage 25. During the course of some operations therefore, by suitably moving the deviator, solid fragments such as bone parts can be collected up, recovered and reused.

## Claims

1. A filtering device comprising:
- one hollow body (11) defining a filtering chamber (14) and around this at least one longitudinal channel (16),
- a first end connection (12) for coupling an aspiration piece to one end of said body,
- a second end connection (13) to connect to an aspiration unit,
- a removable filter (15) placed in said chamber, and wherein:
- the first connection (12) has an entrance anti-chamber (19) that communicates, on the one side, with an entrance passage (20) and, on the other, with an entrance conduit (21) communicating with said filtering chamber (14) and with an entrance compartment (22) that communicates with said longitudinal channel (16) in said body (11), the aspiration piece being connected to the entrance passage (20),
- the second connection (13) has an evacuation passage (25) communicating with an exit conduit (26) communicating with said filtering chamber, and an exit compartment (27) communicating with said longitudinal channel (16) in said body, the evacuation passage being connected to the aspiration unit,
- in the anti-chamber of said first connection a diverter means (29) is located and movable between a first position, in which said entrance passage (20) communicates with said longitudinal channel through the entrance compartment (22), and a second position, in which said entrance passage (20) communicates with said filtering chamber (14) through the entrance conduit (21).

2. The filtering device in accordance with claim 1, wherein said diverter means (29) consists of a tubular piece (30) movable around an oscillation axis (31) and having an inlet (30') permanently in line with said entrance passage (20) and an outlet (30") which in said first position is in line with the entrance compartment (22) and in said second position is positioned in line with the entrance conduit (21).

3. The filtering device in accordance with claim 2, wherein said diverter means (29,30) is pressed and kept in said first position by a spring (32) and is engaged and movable in said second position by a button (33).

4. The filtering device according to the previous claims, wherein the first connection and the second connection are identical in construction, and wherein only the first connection is provided with a diverter means.

5. The filtering device according to claim 4, wherein each first and second connection (12,13) consists of two complementary casings, facing and joined to one another with annular fasteners (17), wherein every connection is fastened to the respective end of the body (11) and features an internal part (12', 13') associated by sealing with the filtering chamber (14) with the interposition of at least one seal (18) and wherein the conduit (21) communicating with the filtering chamber is in said internal part.

6. The filtering device according to claim 1, wherein said diverter means is linearly movable between said first and said second position and features passages for putting alternately into communication the entrance passage with the entrance compartment and with the filtering chamber.
